# EUROPÄISCHE PATENTANMELDUNG

(11) **EP 4 481 445 A1**
(43) Veröffentlichungstag der Anmeldung: **25.12.2024**
(21) Anmeldenummer: 23180925.2
(22) Anmeldetag: 22.06.2023
(51) Int. Cl.: G01T 1/29, G01T 1/24, G01T 7/00

(54) **CT-DETEKTORMODUL UND CT-GERÄT**

(71) Anmelder: Siemens Healthineers AG, 91301 Forchheim (DE)
(72) Erfinder: Ellwood, Robert John, 91056 Erlangen (DE); Labayen de Inza, Miguel, 91301 Forchheim (DE); Wrege, Jan, 91056 Erlangen (DE); Wölfel, Stefan, 91077 Dormitz (DE)
(74) Vertreter: Siemens Healthineers Patent Attorneys

(57) **Zusammenfassung**

Die Erfindung betrifft CT-Detektormodul (2) und ein CT-Gerät. Das CT-Detektormodul umfasst zumindest ein Röntgenkonverterelement (6), welches eine Oberseite und eine Unterseite aufweist, wobei das Röntgenkonverterelement (6) an seiner Oberseite (O) eine Röntgendetektorschicht (8,9) aufweist und an seiner Unterseite auf einem wärmeleitenden Modulträger (4) befestigt ist. Das Röntgenkonverterelement (6) steht zu zumindest einem Metallelement (12) in wärmeleitendem Kontakt, und dass das zumindest eine Metallelement (12) in wärmeleitendem Kontakt zu dem Modulträger (4) steht.

## Beschreibung

Die Erfindung betrifft ein CT-Detektormodul, sowie ein CT-Gerät.

Moderne Computed-Tomography (CT) Geräte haben eine Gantry mit einem drehbaren Rahmen, an dem unter anderem die Röntgenquelle sowie ein Detektormodul zum Erfassen der Röntgenstrahlung angeordnet sind. Ein derartiges CT-Detektormodul umfasst in der Regel ein Röntgenkonverterelement, welches eine Röntgensensorschicht und ggf. eine darunter angeordnete Schicht mit A/D (analog-zu-digital) Wandlern aufweist. Die elektronische Integration war in letzter Zeit ein wichtiger Trend bei den Röntgenkonverterelementen. Ziel dabei war es, die Länge des analogen Pfades zwischen der analogen Röntgensensorschicht und den A/D-Wandlern, welche üblicherweise als ASICs (Application-Specific Integrated Circuit) realisiert werden, zu reduzieren. Bei integrierenden Röntgenkonvertern wird die analoge Röntgensensorschicht durch eine geeignete Sensorschicht, beispielsweise einem Szintillator, in Kombination mit einer Fotodiode gebildet, während bei zählenden (counting) Röntgenkonvertern ein direkt-konvertierender Halbleitersensor verwendet wird. Ein als ASIC ausgebildeter A/D-Wandler erzeugt dann das digitale Ausgangssignal. In beiden Fällen bringt eine Integration der ASICs in einen kompakten Aufbau, insbesondere Stapelaufbau, zusammen mit der analogen Röntgensensorschicht eine bedeutende Wärmequelle näher an die Röntgensensorelemente der Röntgensensorschicht selbst.

Da die Röntgensensorelemente sehr sensibel auf Wärmeschwankungen reagieren, ist das Wärmemanagement eine entscheidende Aufgabe bei der Entwicklung eines modernen CT-Detektormoduls. Dabei sind besondere Herausforderungen des Wärmemanagements, die Betriebstemperatur des CT-Detektormoduls stabil zu halten, Temperaturgradienten zwischen den benachbarten Röntgenkonverterelementen zu vermeiden und auch innerhalb jedes Röntgenkonverterelements die Temperaturgradienten zu reduzieren. Diese Herausforderungen sind bei zählenden (counting) Röntgenkonvertern sogar noch wichtiger, da die direkt konvertierenden Halbleitersensoren zusätzliche Wärmequellen sind und ihre Sensorleistung gleichzeitig sehr empfindlich auf thermische Veränderungen reagiert.

Derzeit wird das Wärmemanagement dadurch gewährleistet, dass eine thermische Schnittstelle zwischen dem Röntgenkonverterelement und dem Metallrahmen des CT-Detektormoduls durch einen wärmeleitenden Klebstoff oder eine wärmeleitende Paste hergestellt wird. Dadurch ist es aber nicht möglich, eine definierte Wärmemenge zu transportieren, da die Güte der thermischen Schnittstelle von verschiedenen, nicht gut zu beeinflussenden Faktoren abhängt, insbesondere der Dicke des Spalts zwischen dem Röntgenkonverter und dem Metallrahmen, sowie der genauen Verteilung der Wärmeleitpaste.

Es ist daher eine Aufgabe der Erfindung, ein CT-Detektormodul bereitzustellen, bei welchem die Wärmeenergie so effektiv wie möglich vom Röntgenkonverterelement abgeleitet werden kann, insbesondere in einen Modulträger. Es ist eine weitere Aufgabe der Erfindung, ein CT-Detektormodul bereitzustellen, bei welchem die Temperaturgradienten innerhalb eines Röntgenkonverterelements sowie zwischen benachbarten Röntgenkonverterelementen gegenüber dem Stand der Technik reduziert wird.

Diese Aufgabe löst die Erfindung mit einem CT-Detektormodul nach Anspruch 1 sowie einem CT-Gerät nach Anspruch 13.

Die Erfindung stellt ein CT-Detektormodul bereit, welches zumindest ein Röntgenkonverterelement umfasst, welches eine Oberseite und eine Unterseite aufweist, wobei das Röntgenkonverterelement an seiner Oberseite eine Röntgendetektorschicht aufweist und an seiner Unterseite auf einem wärmeleitenden Modulträger befestigt ist. Das Röntgenkonverterelement steht zu zumindest einem Metallelement in wärmeleitenden Kontakt, und das zumindest eine Metallelement steht in wärmeleitendem Kontakt zu dem Modulträger.

In dem erfindungsgemäßen CT-Detektormodul wird also der thermische Kontakt zwischen dem Röntgenkonverterelement und dem Modulträger durch Metall-zu-Metall Kontakte hergestellt, welche daher eine hohe Wärmeleitfähigkeit aufweisen. Der Metall-zu-Metall Kontakt wird zwischen dem Röntgenkonverterelement und einem Zwischenelement aus Metall, dem Metallelement, hergestellt, und ferner zwischen diesem Metallelement und dem Modulträger. Bei dem Modulträger kann es sich insbesondere um einen Metallrahmen mit Konvektionskühlerelementen wie beispielsweise Kühlrippen handeln. Durch die Verwendung des zumindest einen Metallelementes ist es möglich, die thermische Schnittstelle zwischen dem Röntgenkonverterelement und dem Modulträger genau zu definieren, da die metallischen Kontaktstellen genau festlegbar sind. Die Metall-zu-Metall wärmeleitenden Kontakte sind vorzugsweise lückenlos ausgebildet, um eine möglichst hohe Wärmeleitung zu erreichen. Die Metall-zu-Metall Kontakte können hergestellt werden, nachdem das CT-Detektormodul mit der notwendigen Genauigkeit auf dem Modulträger befestigt, beispielsweise aufgeklebt, wurde, insbesondere mit UV-Kleber. In vielen Fällen ist es möglich, den thermischen Kontakt weiter durch beispielsweise Wärmeleitpasten oder -pads oder Klebstoffe oder Lotmaterialien zu verbessern, um den thermischen Kontakt zu erhöhen.

Gemäß einer Ausführungsform handelt es sich bei dem Metallelement nicht um ein Lot. Insbesondere handelt es sich bei dem Metallelement um ein formstabiles Element, welches beispielsweise die Form einer Feder, eines Blechs, eines Stifts, einer Schraube, eines Federelements oder dergleichen annehmen kann.

In vorteilhaften Ausführungsformen der Erfindung wird die Wärme von jedem Röntgenkonverterelement nicht nur an einer Stelle, sondern an mehreren Stellen der Unterseite und/oder flächig abgeleitet. Mit "flächig" ist hier gemeint, dass der Kontakt nicht nur punktuell ist, sondern über eine größere Fläche der Unterseite des Röntgenkonverterelements stattfindet, beispielsweise über eine Fläche von 10%-90%, vorzugsweise 20%-80%, stärker bevorzugt 30%-60% der Unterseite des Röntgenkonverterelements.

Das Röntgenkonverterelement weist auf seiner Oberseite eine Röntgendetektorschicht auf. Diese ist der Röntgenquelle zugewandt und ist dazu ausgelegt, die Röntgenstrahlen zu detektieren. Die Röntgendetektorschicht kann eine direktkonvertierende (Halbleiter-)Röntgensensorschicht, beispielsweise aufweisend CdTe, CdZnTe, CdTeSe, CdZnTeSe, CdMnTe, GaAs, Si oder Ge als Halbleitermaterial, umfassen. Die Röntgendetektorschicht kann auch eine geeignete Sensorschicht, welches Röntgenstrahlung in Licht wandelt, und optisch gekoppelte Fotodioden, insbesondere ein oder mehrere Fotodiodenarrays umfassen. Als Material wird dabei häufig Szintillatormaterial, beispielsweise GOS (Gd2O2S), CsJ, YGO oder Lu-TAG, eingesetzt. Die Röntgendetektorschicht kann außerdem eine Schicht mit Analog-zu-Digital-Wandlern umfassen, auf welche die Röntgensensorschicht aufgebracht ist, wobei die A/D-Wandlerschicht in einem oder mehreren ASICs realisiert werden kann. Die Röntgendetektorschicht kann auf einer Basisplatte, auch Substrat genannt, beispielsweise einer Leiterplatte oder einem Keramik- oder Glassubstrat, aufgebracht sein, welches dann die Unterseite des Röntgenkonverterelements bildet. Das Röntgenkonverterelement ist an seiner Unterseite auf einem wärmeleitenden Modulträger befestigt.

Der Modulträger ist insbesondere aus Metall, z.B. aus Aluminium-Druckguss. Der Modulträger kann zur mechanischen Fixierung eines jeweiligen Detektormoduls in einem das Detektormodul umfassenden CT-Geräts ausgebildet sein. Dazu können entsprechende Fixierungsmittel und Ausrichtungsmittel am Modulträger vorgesehen sein. Der Modulträger ist vorzugsweise als metallischer Kühlkörper ausgebildet, welcher für die Temperaturstabilisierung des Detektormoduls und die Abfuhr von Wärme aus dem Röntgenkonverterelement ausgebildet ist. Beispielsweise kann der Modulträger mit geeigneten Kühlrippen ausgestattet sein. Das CT-Detektormodul ist typischerweise Teil einer rotierenden Gantry eines CT-Geräts.

Das Röntgenkonverterelement kann durch einen Klebstoff, beispielsweise einen UV-Kleber, auf dem Modulträger befestigt sein, wobei andere Befestigungsmittel wie beispielsweise Schrauben auch möglich sind. Es können auch mehrere Röntgenkonverterelemente auf dem Modulträger befestigt sein, sodass dieser damit durch die Aneinanderreihung der Röntgenkonverterelemente "ausgekachelt" ist.

Die Erfindung zeichnet sich ferner dadurch aus, dass über ein neuartiges Metallelement die im Röntgenkonverterelement und insbesondere in der Röntgendetektorschicht erzeugte Wärme effizient in den Modulträger ableitet wird.

Gemäß einer bevorzugten Ausführungsform ist die Unterseite des Röntgenkonverterelements, welches in wärmeleitendem Kontakt zu dem mindestens einen Metallelement steht, zumindest zum Teil mit einem metallischen Belag versehen. Dadurch ist es möglich, die Wärmeleitung auf den Modulträger zu verbessert und gegebenenfalls einen großflächigen Kontakt zum Metallelement herzustellen, wodurch die Wärmeleitung auf den Modulträger noch weiter verbessert wird. Der metallische Belag kann beispielsweise durch eine Beschichtung mit einem gut wärmeleitenden Metall, z.B. Gold, Silber oder Kupfer, gebildet sein. Der metallische Belag kann ein sogenanntes "metallized thermal pad", ein metallisiertes Wärmeleitpad, sein. Der wärmeleitende Belag kann einen Teil oder auch die gesamte Unterseite des Röntgenkonverterelements bedecken, insbesondere ist er an der Stelle vorgesehen, an denen das Metallelement am Röntgenkonverterelement anliegt. Der metallische Belag kann durch metallisierte Durchgangslöcher in einer Basisplatte und/oder der A/D-Wandlerschicht der Röntgendetektorschicht mit der der A/D-Wandlerschicht und/oder der Röntgensensorschicht verbunden sein, damit die Wärme von dort in den metallischen Belag abgeleitet werden kann. Dies wird auch als thermische Via-Technologie bezeichnet. Bei den metallisierten Durchgangslöchern kann es sich beispielsweise um Kontaktlöcher in der Basisplatte handeln.

Gemäß einer Ausführungsform weist das zumindest eine Metallelement ein flaches Metallelement, insbesondere Metallblech auf, welches in einem ersten Bereich an der Unterseite des Röntgenkonverterelements anliegt und in einem zweiten Bereich am Modulträger anliegt. Die Ausführung als flaches Metallelement ist günstig in der Herstellung und gewährleistet eine sehr gute Wärmeleitung, insbesondere wenn das Metallblech an flächigen Bereichen auf seine Ober- und/oder Unterseite an den zu verbindenden Elementen anliegt. Das flache Metallelement kann z.B. zwei Schenkel aufweisen, von denen der eine Schenkel an der Unterseite des Röntgenkonverterelements anliegt und der andere Schenkel am Modulträger anliegt, wobei die beiden Schenkel z.B. einen Winkel von etwa 90° ausmachen können. Das Metallblech kann also L-förmig oder T-förmig im Querschnitt sein. Das Metallelement kann auch ein Profilelement sein, welches ebenfalls zwei Schenkel aufweisen kann. Der zweite Schenkel kann in einer Ausnehmung des Modulträger angeordnet und gegebenenfalls dort befestigt sein, z.B. durch ein Befestigungselement wie eine Schraube. Die Ausnehmung kann beispielsweise ein Spalt sein, in den der zweite Schenkel des Metallelements eingeschoben wird. In einer anderen Ausführungsform bildet das Metallelement, insbesondere Metallblech, eine Scheibe, welche zwischen der Unterseite des Röntgenkonverterelements und der Oberseite des Modulträgers eingelegt ist und somit mit seiner Oberseite mit dem Röntgenkonverterelement in wärmeleitendem Kontakt steht und mit seiner Unterseite mit dem Modulträger. Auch bei dieser Ausführungsform können Befestigungsmittel oder Federelemente vorgesehen sein, welche dafür sorgen, dass der Kontakt hergestellt wird. Ein solches Blech kann auch nur eines von mehreren wärmeleitenden Metallelementen sein, insbesondere können auch die Befestigungsmittel aus Metall sein und somit einen Teil der Wärmebrücke bilden.

Gemäß einer Ausführungsform weist das zumindest eine Metallelement ein Federelement auf, welches durch eine Rückstellkraft an die Unterseite des Röntgenkonverterelements gedrückt wird. Das Federelement kann aus einem Metallblech gebildet sein und die Eigenschaften aufweisen, die oben beschrieben wurden. Beispielsweise kann ein L-förmig vorgebogenes Metallblech mit einer Vorspannung zwischen das Röntgenkonverterelement und dem Modulträger so angeordnet werden, dass es gegen die Unterseite des Röntgenkonverterelements drückt. Die Ausführungsform, in dem das Metallelement ein Metallblech bzw. ein Federelement aufweist, werden besonders vorteilhaft mit der Ausführungsform kombiniert, bei welchen die Unterseite des Röntgenkonverterelements zumindest zum Teil mit einem metallischen Belag versehen ist. Der mit dem Belag versehene Teil kann beispielsweise 20%-80%, vorzugsweis 40%-60% der Unterseite des Röntgenkonverterelements ausmachen.

Das Federelement kann als Einsatz in den Modulträger eingesetzt sein, insbesondere in eine Ausnehmung von diesem. Beim Zusammenbauen des CT-Detektormoduls kann das Röntgenkonverterelement in einem ersten Schritt auf den Modulträger geklebt werden, vorzugsweise mit einer möglichst hohen Positionsgenauigkeit. Dabei kann ein schnellhärtender Klebstoff wie beispielsweise UV-Klebstoff verwendet werden. Vorzugsweise ist beim Kleben das Federelement oder Metallblech bereits in dem Modulträger eingesetzt, es kann in bestimmten Ausführungsformen jedoch auch nach dem Klebevorgang von der Seite eingeschoben werden.

Der Metall-zu-Metall Oberflächenkontakt kann dadurch erhöht werden, dass die jeweils anliegenden Bereiche aneinander gepresst werden, insbesondere durch ein Befestigungselement oder ein Element mit einer Rückstellkraft, z.B. eine Feder. Beispielsweise kann eine Schraube, z.B. eine Madenschraube, in eine Bohrung in einen Modulträger eingeschraubt werden, die bis zum zweiten Bereich des Metallblechs bzw. des Federelements reicht. Dadurch kann dieser Bereich an den Modulträger gepresst werden. Darüber hinaus findet auch eine Wärmeleitung durch die Schraube selber statt. Ebenso kann auch eine weitere Bohrung bis zum ersten Bereich des Metallelements reichen. Eine durch diese Bohrung eingesetzte Madenschraube kann an den ersten Bereich des Metallelements an die Unterseite des Röntgenkonverterelements andrücken.

Alternativ ist es auch möglich, das Metallelement an seinen flächigen Kontaktbereichen, an denen es mit der Unterseite des Röntgenkonverterelements und dem Modulträger Kontakt haben soll, durch beliebige Befestigungsmittel thermisch mit diesen zu verbinden. Die Befestigungsmittel können beispielsweise Schrauben, Nieten, Kolbenfedern, ein Lot oder auch ein reaktives Lot sein. Unter reaktivem Lot versteht man ein Verfahren, bei dem auf die zu verbindenden Flächen ein Multischichtsystem, welches in der Regel aus zwei verschiedenen Metallen besteht, welche abwechselnd übereinander beschichtet sind, beispielsweise Nickel/Aluminium, Al/Ti, oder Cu/Ag. Die zum Schmelzen des Lots notwendige Hitze wird durch eine sich selbst propagierende exotherme Reaktion des Multischichtsystems ausgelöst. Die Reaktion wird in der Regel durch einen Energieimpuls gezündet, z.B. durch hohe Temperatur, mechanischem Druck, einen elektrischen Funken oder Laserpuls. In der Erfindung ist es also möglich, entsprechende reaktive Lotschichten auf die Bereiche des Metallelements aufzubringen, an denen dieses am Röntgenkonverterelement und am Modulträger anliegt. Wenn das CT-Detektormodul soweit zusammengesetzt ist, dass die Röntgenkonverterelemente an der vorgesehenen Position auf dem Modulträger geklebt sind, kann die Reaktion gezündet und somit eine innig metallische Verbindung zwischen dem Metallelement und dem Röntgenkonverterelement sowie dem Modulträger hergestellt werden. Auch bei dieser Ausführungsform erweist sich der metallische Belag auf der Unterseite des Röntgenkonverterelements als vorteilhaft. Alternativ kann das reaktive Lot auch bereits vor dem Montieren der Röntgenkonverterelemente gezündet werden.

Gemäß einer Ausführungsform ist das zumindest eine Metallelement durch ein Befestigungsmittel an dem Modulträger befestigt. Vorzugsweise ist das Befestigungsmittel aus Metall und bildet somit ebenfalls einen Teil der Wärmebrücke bzw. des Metallelements. Das Befestigungsmittel kann insbesondere eine Schraube, Niete, Klemme, ein Stift oder eine Kolbenfeder sein, ein Lot oder auch ein reaktives Lot. Vorzugsweise ist das Befestigungsmittel eine Schraube, welche in einer Bohrung im Modulträger eingeführt ist und gegen das Metallelement drückt bzw. das Metallelement gegen einen Teil des Modulträgers presst, um dieses zu befestigen. Insbesondere kann es sich um Metallschrauben, z.B. Madenschrauben, handeln.

Gemäß einer Ausführungsform umfasst das zumindest eine Metallelement zumindest ein Befestigungselement, welches am Modulträger festgelegt ist und gegen einen metallischen Belag auf der Unterseite des Röntgenkonverterelements oder gegen einen an der Unterseite des Röntgenkonverterelements angeordnetes flächiges Metallelement vorgespannt ist. In dieser Ausführungsform ist es möglich, dass die Befestigungselemente die Haupt-Wärmeleiter darstellen. Die Befestigungselemente können wie oben beschrieben ausgebildet sein, also als Schraube, Niete, Klemme, Stift oder Kolbenfeder. In einer Ausführungsform sind die Befestigungselemente Schrauben, welche in entsprechende Gewindebohrungen im Modulträger eingeschraubt sind. Insbesondere kann es sich dabei um Bohrungen handeln, die von unten durch den Modulträger durch diesen hindurch bis zur Unterseite des Röntgenkonverterelements reichen. Die Bohrungen können z.B. senkrecht auf die Unterseite des Röntgenkonverterelements stehen. Wenn Metallschrauben in diese Bohrungen eingedreht werden, können sie bis zur Unterseite des Röntgenkonverterelements reichen und somit den thermischen Kontakt herstellen. Bei der Herstellung des CT-Detektormoduls kann das Röntgenkonverterelement in einem ersten Schritt auf den Modulträger geklebt werden. Vorzugsweise befindet sich auf der Unterseite des Röntgenkonverterelements ein metallischer Belag, der vorzugsweise durch thermische Via-Technologie mit den Wärmequellen, insbesondere der Röntgendetektorschicht, verbunden ist. Die eingeführten Metallschrauben oder auch Schrauben mit metallischen Kolbenfedern wirken dann als Wärmeleitkomponenten zwischen der Unterseite des Röntgenkonverterelements und dem Modulträger. Metall-zu-Metall Oberflächenkontakte können dadurch erreicht werden, dass die Schrauben gegen die Unterseite des Röntgenkonverterelements, insbesondere gegen den metallischen Belag, vorgespannt sind.

Gemäß einer Ausführungsform ist das zumindest eine Metallelement in einer Ausnehmung im Modulträger eingesetzt oder an einer Seitenwand des Modulträgers befestigt. Die Seitenwand ist insbesondere eine zur Ober- und Unterseite des Röntgenkonverterelement zumindest im Wesentlichen senkrecht stehende Wand. Die Ausnehmung kann insbesondere ein flächiges Metallelement wie ein Metallblech beherbergen. An einer Seitenwand des Modulträgers kann beispielsweise ein Metallelement in Form eines Metallblocks befestigt sein, beispielsweise durch ein Befestigungsmittel wie eine Schraube, und dieser Metallblock kann ebenfalls von unten gegen die Unterseite des Röntgenkonverterelements anliegen bzw. gegen dieses drücken. Hierbei kann das Röntgenkonverterelement wiederum in einem ersten Schritt auf den Modulträger geklebt werden. Die Unterseite des Röntgenkonverterelements kann einen metallischen Belag aufweisen, welcher durch thermische Via-Technologie mit den Wärmequellen verbunden sein kann. Ein Metallelement, welches beispielsweise als ein Metalleinsatz bzw. ein Metallblock ausgebildet ist, kann die Unterseite des Röntgenkonverterelements thermisch mit dem Modulträger verbinden. Dabei wird der Metall-zu-Metall Kontakt dadurch hergestellt, dass der Metalleinsatz gegen die Unterseite des Röntgenkonverterelements gedrückt wird und das Metallelement dann in dieser Position im Modulträger festgeklemmt wird, beispielsweise durch ein Befestigungsmittel wie eine Schraube. Alternativ können auch andere Verbindungstechnologien verwendet werden, um den Metallblock in seiner finalen Position zu halten.

Gemäß einer Ausführungsform ist das zumindest eine Metallelement in einer Ausnehmung im Modulträger eingesetzt oder an einer Seitenwand des Modulträgers befestigt. Dadurch, dass das Metallelement in einer Ausnehmung eingesetzt ist oder an die Seitenwand anliegt, ist eine besonders hohe Wärmeleitung möglich. Bei der Seitenwand des Modulträgers kann es sich um eine Wand handeln, welche senkrecht zu der Unterseite des Röntgenkonverterelements verläuft. Das Metallelement kann z.B. quaderförmig ausgestellt sein, sodass es mit zwei nebeneinanderliegenden Seiten Kontakt sowohl zur Unterseite des Röntgenkonverterelements und zum Modulträger hat. Die Ausnehmung kann ein Schlitz sein, in dem ein flächiges Metallelement eingesetzt ist, beispielsweise ein Schenkel eines Metallblechs.

Gemäß einer Ausführungsform weist das zumindest eine Metallelement ein Reaktivlot auf, welches die Unterseite des Röntgenkonverterelements wärmeleitend mit dem Modulträger verbindet. Es ist möglich, dass das Reaktivlot das Metallelement bildet. Es ist aber auch möglich, dass das Reaktivlot zusätzlich zu einem flächigen Metallelement, z.B. einem Metallblech, verwendet wird, welches zwischen das Röntgenkonverterelement und dem Modulträger eingelegt wird. In der erstgenannten Ausführungsform wird die wärmeleitende Paste mit der Lottechnologie ersetzt. Alternativ kann statt des Reaktivlots ein normales Lot verwendet werden, welches die Unterseite des Röntgenkonverterelements wärmeleitend mit dem Modulträger verbindet.

In dieser Ausführungsform kann die metallische Schnittstelle dadurch realisiert werden, dass ein lokalisierter, selektiver Lotprozess durchgeführt wird, welche die Unterseite des Röntgenkonverterelements und, falls vorhanden, ein dort angeordneter metallischer Belag, mit der Metalloberfläche des Modulträgers verbindet. Bei dieser Ausführungsform kann das gesamte Röntgenkonverterelement in einem ersten Schritt wiederum mit hoher Positionsgenauigkeit auf den Modulträger geklebt werden. Auf der Unterseite des Röntgenkonverterelements sind vorzugsweise ein oder mehrere Stellen mit einem metallischen Belag versehen, die vorzugsweise mit den Wärmequellen durch thermische Via-Technologie verbunden sein können. Ein selektiver Lotprozess kann mittels eines Reaktivlots umgesetzt sein. Wenn das Reaktivlot gezündet wird, wird eine Lotverbindung direkt als wärmeleitendes Element zwischen der Unterseite des Röntgenkonverterelements und dem Modulträger implementiert. Das Zünden des Reaktivlots kann durch die bekannten Zündtechnologien wie Laser oder Erhitzen (z.B. durch Funken oder Flammen) ausgelöst werden. Alternativ kann statt des Reaktivlots ein normales Lot verwendet werden, welches entweder nach dem Verkleben in den Spalt zwischen der Unterseite des Röntgenkonverterelements bzw. des dort angeordneten metallischen Belag und der Oberseite des Modulträgers eingeführt wird. Alternativ kann das normale Lot auch bereits vor dem Klebeschritt in diesen Spalt eingefügt sein und dann lediglich durch konventionelle Wärmetechnologie wie Laser oder (weniger bevorzugt) Flammen erhitzt werden, um die metallische Verbindung herzustellen.

Gemäß einer Ausführungsform weist das Röntgenkonverterelement an seiner Unterseite eine Basisplatte auf, welche eine Ausnehmung aufweist, durch welche das zumindest eine Metallelement direkt mit der Röntgendetektorschicht in wärmeleitendem Kontakt steht. Bei dieser Ausführungsform hat die Basisplatte des Röntgenkonverterelements eine durchgehende Ausnehmung, insbesondere in der Mitte. Es kann beispielsweise eine rechteckige, runde oder ovale Ausnehmung sein, welches die Randelemente stehen lässt. Insbesondere ist die Ausnehmung als ein Durchgangsloch ausgebildet, sodass das Metallelement durch die Ausnehmung hindurch direkt bis zur Unterseite der Röntgendetektorschicht geführt werden kann. Auf diese Weise kann die Wärme noch effizienter abgeführt werden. Das Metallelement kann wie hierin beschrieben ausgebildet sein, also beispielsweise als Federelement, als Metallblech, Metallblock, Schraube, etc. Die Unterseite der Röntgendetektorschicht ist vorzugsweise zumindest teilweise mit einem metallischen Belag versehen, beispielsweise in Form einer Beschichtung oder eines metallized thermal pads, welcher wiederum mit der Röntgendetektorschicht bzw. der Röntgensensorschicht durch metallisierte Durchgangslöcher verbunden sein kann. Damit wirkt das Metallelement als Wärmebrücke zwischen der Röntgendetektorschicht und dem Modulträger. Die Verbindung zwischen dem Metallelement und der Unterseite der Röntgendetektorschicht kann insbesondere auf die hier in Bezug zu den anderen Ausführungsformen beschriebenen Weisen hergestellt werden. Beispielsweise können Schrauben die Metallflächen aneinander pressen. Alternativ können Verbindungstechnologien wie reaktives Löten vorab oder als finaler Schritt eingesetzt werden.

Gemäß einer Ausführungsform weist das Röntgenkonverterelement an seiner Unterseite eine Basisplatte auf, wobei die Basisplatte zumindest in dem Bereich, in welchem sie mit dem zumindest einem Metallelement in wärmeleitender Verbindung steht, wärmebrückend zwischen der Unterseite der Basisplatte und der Röntgendetektorschicht aufweist. Die Wärmebrücken können beispielsweise metallisierte Durchgangslöcher sein, welche dafür sorgen, dass der Wärmefluss zwischen der Röntgendetektorschicht und dem Modulträger verbessert wird. Die Wärmebrücken können auch durch zusätzliche Metallschichten gebildet werden.

Die Erfindung stellt ein robustes und hocheffizientes Wärmemanagementkonzept für die Integration von Röntgenkonverterelementen in CT-Detektormodulen bereit, indem konsequent lückenlose Metall-zu-Metall Kontakte eingesetzt werden. Die Wärmeleitfähigkeit wird daher gegenüber den bekannten Lösungen mit thermischen Kasten oder thermischen Klebstoffen erheblich verbessert. Dadurch, dass Lücken zwischen den einzelnen Elementen des CT-Detektormoduls vermieden bzw. diese Lücken mit dem Metallelement überbrückt werden, wird der thermische Widerstand verringert. Dadurch kann eine verbesserte Temperaturhomogenität über das gesamte CT-Detektormodul bei gleichzeitig verringerter Temperatur erreicht werden. Somit wird die Signalleistung und Stabilität verbessert.

Gemäß einer Ausführungsform weist das Röntgenkonverterelement an seiner Unterseite eine Basisplatte auf, welche plattierte Durchgangslöcher aufweist, welche Wärmebrücken zwischen der Unterseite der Basisplatte und der Röntgendetektorschicht bilden. Dadurch wird die Wärmeleitung weiter verbessert.

Gemäß einer Ausführungsform weist der Modulträger Kühlrippen auf. Dies ist sinnvoll, um die in den Modulträger eingeleitete Wärme an die Umgebungsluft abzugeben. Somit wird die Temperatur des CT-Detektormoduls weiter verringert.

Die Erfindung ist ferner auf ein CT-Gerät gerichtet, welches zumindest eins der erfindungsgemäßes CT-Detektormodul, insbesondere eine Mehrzahl an entlang einer Rotationsrichtung aneinandergereihte CT-Detektormodule, aufweist. Alle mit Bezug auf das CT-Detektormodul genannten Ausführungsformen und Vorteile sind auch auf das CT-Gerät anwendbar und umgekehrt. Das CT-Detektormodul ist insbesondere Teil einer rotierenden Gantry des CT-Geräts. Das CT-Gerät kann weitere übliche Komponenten aufweisen, wie z.B. eine Röntgenquelle, eine Patientenliege usw.

Die Erfindung wird nun anhand von Ausführungsbeispielen mit Bezug auf die beiliegenden Zeichnungen näher erläutert.

In den Zeichnungen zeigen:
- Fig. 1: einen schematischen Querschnitt durch ein CT-Gerät gemäß einer Ausführungsform der Erfindung;
- Fig. 2: einen schematischen Querschnitt in Längs- und Querrichtung durch ein CT-Detektormodul gemäß Stand der Technik;
- Fig. 3: einen Querschnitt in Längs- und Querrichtung durch ein CT-Detektormodul gemäß einer ersten Ausführungsform der Erfindung;
- Fig. 4: einen Querschnitt in Längs- und Querrichtung durch ein CT-Detektormodul gemäß einer zweiten Ausführungsform der Erfindung;
- Fig. 5: einen Querschnitt in Längs- und Querrichtung durch ein CT-Detektormodul gemäß einer dritten Ausführungsform der Erfindung;
- Fig. 6: einen Querschnitt in Längs- und Querrichtung durch ein CT-Detektormodul gemäß einer vierten Ausführungsform der Erfindung;
- Fig. 7: einen Querschnitt in Längs- und Querrichtung durch ein CT-Detektormodul gemäß einer fünften Ausführungsform der Erfindung;
- Fig. 8: einen Querschnitt in Längs- und Querrichtung durch ein CT-Detektormodul gemäß einer sechsten Ausführungsform der Erfindung.

Gleiche Teile sind in den Figuren mit gleichen Bezugszeichen gekennzeichnet.

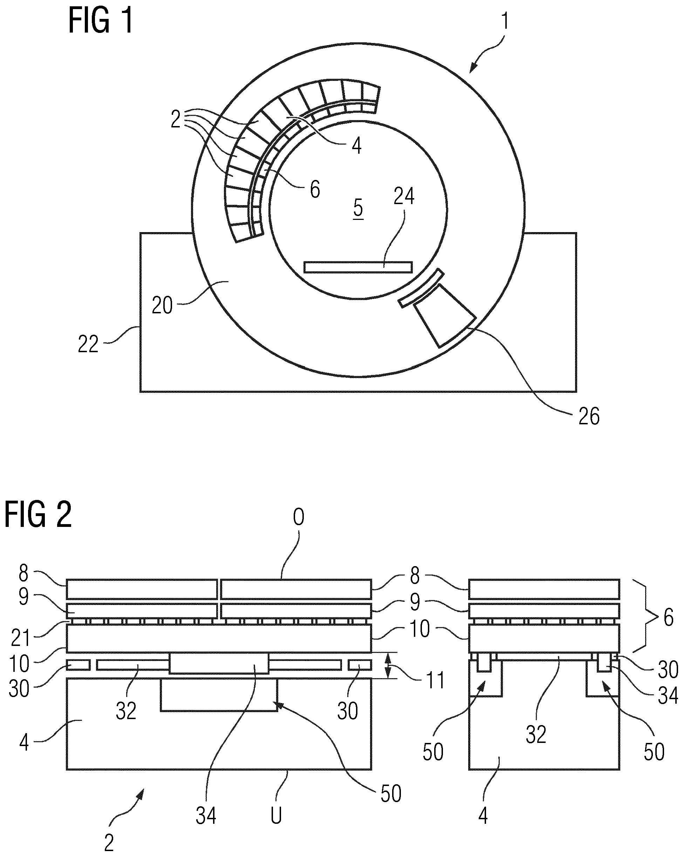

Fig. 1 zeigt einen schematischen Querschnitt durch ein CT-Gerät 1 gemäß einer Ausführungsform der Erfindung. Demnach ist eine rotierende Gantry 20 in einem Geräterahmen 22 gelagert. Die Gantry 20 ist dazu ausgelegt, um einen Untersuchungsraum 5 mit einer Liege 24 zu rotieren, auf der ein Patient gelagert werden kann. Innerhalb der rotierenden Gantry 20 ist eine Mehrzahl an Detektormodulen 2 angeordnet, welche jeweils einen Modulträger 4 umfassen, dessen dem Untersuchungsraum 5 zugewandte Seite mit Röntgenkonverterelementen 6 ausgekachelt ist. Auf der den CT-Detektormodulen 2 gegenüberliegenden Seite befindet sich die Röntgenquelle 26.

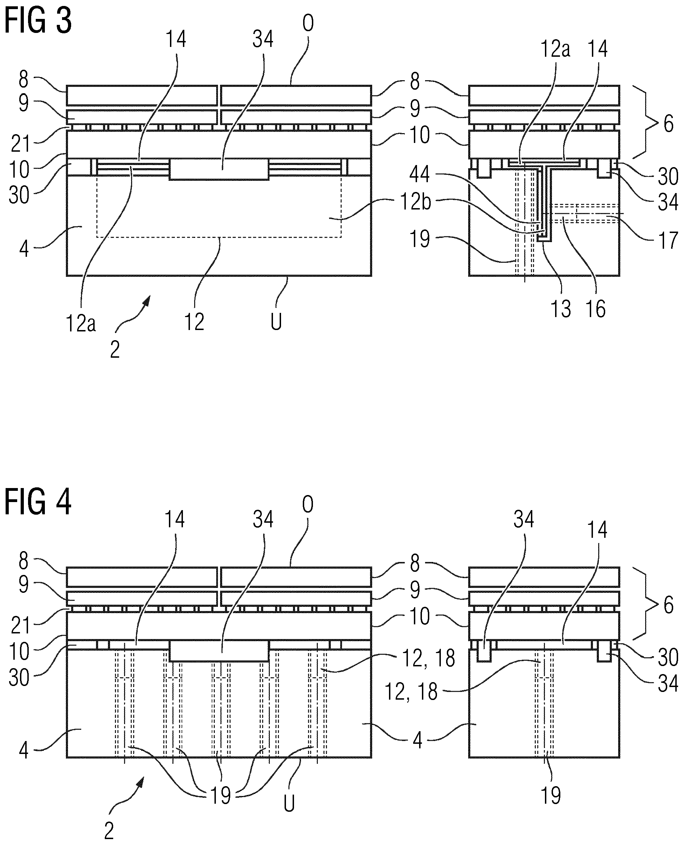

Die folgenden Bilder zeigen jeweils einen schematischen Querschnitt durch ein CT-Detektormodul 2, und zwar auf der linken Seite einen Schnitt in Längsrichtung und auf der rechten Seite einen Schnitt in Querrichtung. Die in den folgenden Figu- ren mit O bezeichnete Oberseite des Detektormoduls ist die Seite, die dem Untersuchungsraum 5 zugewandt ist, die mit U bezeichnete Unterseite ist davon abgewandt. Fig. 2 zeigt auf der linken Seite die Schnittdarstellung schematisch in einem nur teilweise verbundenen Zustand zur Veranschaulichung der Klebeverbindung, wohingegen die rechte Seite im verbundenen Zustand dargestellt ist. Wie in Fig. 2 zu sehen ist, weist das Detektormodul 2 jeweils einen wärmeleitenden Modulträger 4 auf, der insbesondere aus Metall gefertigt ist. Dabei kann es sich beispielsweise um ein Gussteil handeln, z.B. aus einer Aluminiumlegierung. Der Modulträger 4 kann neben den Röntgenkonverterelementen 6 auch die Stromversorgung tragen und auch Kühlrippen aufweisen, um die aufgenommene Wärme effektiv abzuführen. Das Röntgenkonverterelement 6 weist an der Oberseite O eine Röntgendetektorschicht 8,9 auf. Die Röntgendetektorschicht umfasst die Röntgensensorschicht 8, welche z.B. aus CdTe gefertigt sein kann. Darunter liegt eine Schicht aus A/D-Wandlern 9, beispielsweise eine A/Delektrische-Sensorenschicht. Diese Abschnitte 9 können als ASIC ausgebildet sein. Die Röntgensensorschicht 8 und die A/D-Wandlerschicht 9 sind über elektrisch leitende Verbindungen, beispielsweise Lotverbindungen, miteinander verbunden. Zwischenräume können außerdem mittels eines sogenannten "Underfill", also einem Füllmaterial, befüllt sein. Das Füllmaterial kann beispielsweise eine Epoxid-Verbindung, ein Kunststoffmaterial, einen Verbundwerkstoff oder ein (Prä-)Polymer oder anderes aufweisen. Es kann beispielsweise auch eine Leitklebeverbindung vorgesehen sein. Über eine Schnittstellenschicht 21, in der insbesondere elektrische Anschlüsse zur Weiterleitung der Signale von der A/D-Wandlerschicht vorhanden sind, ist die A/D-Wandlerschicht 9 mit einer Basisplatte 10 verbunden. Die Basisplatte 10 ist beispielsweise eine Leiterplatte 10, die insbesondere als Umverdrahtungsebene fungieren kann und im Folgenden auch als Substrat 10 bezeichnet wird. Die Basisplatte 10 kann auch anderweitig ausgebildet sein, beispielsweise als Keramiksubstrat oder ähnliches. Bei 34 ist schematisch ein Steckverbinder dargestellt, über den die in der Basisplatte 10 verarbeitete Daten abgeführt werden können. In diesem Fall wird in dem Steckverbinder 34 beispielsweise ein Flachbandkabel eingesteckt. Dies kann lediglich an einer Seite des Detektormoduls 2 vorgesehen sein. Hier dargestellt ist eine zweiseitige Anordnung von Steckverbindern 34. Außerdem kann der Modulträger 4 Aussparungen 50 aufweisen, welche eine erleichterte Platzierung der Steckverbindung erlauben. In den folgenden Figuren 3 bis 8 sind die Aussparungen 50 im Modulträger 4 der Übersicht halber nicht mehr dargestellt, können aber in gleicher Weise vorliegen. Neben einer hier gezeigten Umsetzung mittels einem Steckverbinder 34 in Kombination mit Aussparungen 50 kann es auch andere Umsetzungen und insbesondere Anordnungen am Detektormodul 2 geben, welche eine Abführung der Daten von dem Substrat 10 ermöglichen und welche beispielsweise keine Aussparungen 50 notwendig machen. Der Modulträger 4 kann z.B. ein Aluminium-Druckgussteil sein.

Fig. 2 zeigt den Stand der Technik, wonach das Röntgenkonverterelement 6 auf dem Modulträger 4 bei 30 mithilfe eines Klebstoffs, beispielsweise eines UV-Klebers, befestigt ist. Diese Klebestellen 30 können jeweils an den Außenrändern der Röntgenkonverterelemente 6 angeordnet sein. Dazwischen wird eine wärmeleitende Paste oder ein wärmeleitender Klebstoff 32 verwendet, der in dem Klebespalt 11 zwischen dem Substrat 10 und dem Modulträger 4 angeordnet ist. Dies hat jedoch den Nachteil, dass die Menge der wärmeleitenden Paste 32 und die Spaltbreite 11 aufgrund von Fertigungstoleranzen schwanken können und die Spaltfüllung daher eine unterschiedlich starke thermische Verbindung darstellt. Ferner kann die Wärmepaste 32 während der Fertigung teilweise aus dem Spalt 11 herausgedrückt werden, wenn z.B. das Klebervolumen groß und der Spalt klein ist, wodurch sich dann die Wärmeleitung zwischen den Röntgenkonverterelementen 6 und dem Modulträger unkontrolliert verschlechtert. Es können mehrere Röntgenkonverterelemente 6 auf einem Modulträger flächig geklebt sein; man spricht auch davon, dass der Modulträger gekachelt ist. Dabei kann eine Basisplatte 10 sich beispielsweise auch über mehrere Röntgenkonverterelemente 6 erstrecken oder jeweils nur einem zugeordnet sein.

Die Erfindung hat sich das Ziel gesetzt, die Wärmeverbindung zwischen den Röntgenkonverterelementen 6 und dem Modulträger 4 zu verbessern. Dies geschieht insbesondere durch eine Erhöhung und bessere Regulierbarkeit des Wärmetransports über den Klebespalt 11 zwischen dem Röntgenkonverterelement 6 und dem Modulträger 4.

Eine erste Ausführungsform der Erfindung ist in Fig. 3 dargestellt. Hierbei ist zum Einen die Unterseite des Substrats 10 mit einem metallischen Belag 14, beispielsweise in Form einer Beschichtung oder einem metallized thermal pad, versehen. Dieser hat die Aufgabe, die Fläche zu vergrößern, über die Wärme vom Röntgenkonverterelement 6 an den Modulträger 4 abgegeben werden kann und/oder die Wärmeleitung zu verbessern. Der metallische Belag kann auch in mehreren Teilbereichen über die Unterseite des Röntgenkonverterelements verteilt, beispielsweise in Form von mehreren beschichteten Flächenabschnitten oder mehreren thermal pads, angeordnet sein. Es kann sich bei dem metallischen Belag beispielsweise um eine Goldschicht handeln. Die Dicke des metallischen Belags kann z.B. 0,5µm - 20µm, vorzugsweise 1µm - 10µm, betragen. Insbesondere über eine thermische Via-Technologie kann der metallische Belag 14 mit den oberen Schichten 8, 9 des Röntgenkonverterelements in Verbindung stehen. Es kann jedoch auch Ausführungen geben ohne einen solchen metallischen Belag. Dabei kann eine wärmeleitende Verbindung mittels einer thermischen Via-Technologie auch ohne einen metallischen Belag zu einer verbesserten Wärmeleitung von den oberen Schichten 8,9 führen. Darüber hinaus weist das Detektormodul 2 ein neues Metallelement 12 auf, welches, wie auf der rechten Seite veranschaulicht, in einer Ausnehmung 13 in Modulträger 4 eingesetzt ist. Im gezeigten Beispiel handelt es sich um ein flächiges Metallelement in T-Form, welches einen Schenkel 12a aufweist, welcher parallel zur Oberseite der Sensorschicht angeordnet ist und an der Unterseite des Substrats bzw. an dem metallischen Belag anliegt. Der andere Schenkel 12b ist in die Ausnehmung 13 eingesetzt und hat hier Kontakt zum Modulträger 4. Der Modulträger 4 kann, wie auf der rechten Seite veranschaulicht, an der Kontaktfläche zum Metallelement 12 ebenfalls mit einem optionalen metallischen Belag 44 versehen sein, z.B. einer Goldschicht, welche die Wärmeleitfähigkeit noch weiter verbessert. Das Metallelement 12 kann zunächst lose in die Ausnehmung 13 eingelegt werden, es hat dabei zunächst noch Spiel. Um sicherzustellen, dass das Metallelement 12 mit seinem Schenkel 12a gegen die Unterseite des Röntgenkonverterelements 6 und mit seinem Schenkel 12b gegen den Modulträger 4 angedrückt wird, können, wie auf der rechten Seite veranschaulicht, Madenschrauben 16 verwendet werden, welche in entsprechende Gewindebohrungen 17, 19 eingeführt werden können. Die Bohrung 17 reicht beispielsweise von der Seite des Modulträgers 4 bis zur Ausnehmung 13, sodass die darin eingeführte Madenschraube 16 beim Anziehen den Schenkel 12b des Metallelements gleichmäßig und flächig gegen die gegenüberliegende Seite der Ausnehmung 13 und somit gegen den Modulträger 4 drückt. Eine zweite Gewindebohrung 19 führt von der Unterseite des Modulträgers 4 bis zur Unterseite des Substrats 10, sodass eine darin eingeführte Madenschraube (nicht dargestellt) den Schenkel 12a des Metallelements gegen die Unterseite des Substrats bzw. gegen den metallischen Belag drückt. Auch an dieser Stelle ist daher eine gleichmäßige und flächige thermische Ankopplung gewährleistet. Das Metallelement 12 kann auch L-förmig ausgestaltet sein, z.B. als gebogenes Blech. In diesem Fall kann es auch als Federelement ausgestaltet sein, also mit einer Vorspannung, mithilfe derer es gegen die Unterseite des Substrats 10 drückt. Die Vorfixierung und mechanische Ausrichtung des Röntgenkonverterelements 6 auf dem Modulträger 4 geschieht weiterhin durch Klebstoff 30, beispielsweise Klebestellen aus einem UV-Kleber, bei den Ausführungsformen der Figuren 3 bis 8. Es kann jedoch auch eine andere Fixierung umgesetzt sein, beispielsweise mittels Schrauben.

Fig. 4 zeigt eine weitere Ausführungsform, in der das wärmeleitende Metallelement 12 wesentlich durch Befestigungsmittel 18, in diesem Fall Madenschrauben 18, gebildet wird, welche in entsprechende Gewindebohrungen 19 im Modulträger 4 eingeschraubt werden, bis sie gegen die Unterseite des Substrats 10 drücken.

Bei dieser Ausführungsform können die Röntgenkonverterelemente 6 zunächst mit Klebstoff 30 auf den Modulträger 4 aufgeklebt werden. Vorzugsweise ist auch bei dieser Ausführungsform an der Unterseite des Substrats 10 ein metallischer Belag 14 vorhanden, zumindest an den Positionen der Gewindebohrung 19. Nach dem Aufkleben und Aushärten werden Madenschrauben 18 oder auch Schrauben mit metallischen Kolbenfedern in die Bohrungen 19 eingeführt und fungieren dann als Wärmeleitelemente zwischen der Unterseite des Substrats 10 und dem Modulträger 4. Dabei entsteht ein Metall-zu-Metall Kontakt durch Anpressen der Befestigungsmittel 18 gegen den metallischen Belag 14. In Ausführungsvarianten kann auch ein weiteres flächiges Metallelement als Teil des Metall-zu-Metall-Kontaktes zwischen der Unterseite des Röntgenkonverterelements 6 und der Oberseite des Modulträgers 4 eingelegt sein und somit mit seiner Oberseite mit dem Röntgenkonverterelement 6 in wärmeleitendem Kontakt steht und mit seiner Unterseite mit dem Modulträger 4. Die Befestigungsmittel 18 sorgen dann durch Anpressen dafür, dass der Kontakt hergestellt und ein effektiver Wärmetransport in den Modulträger gewährleistet wird.

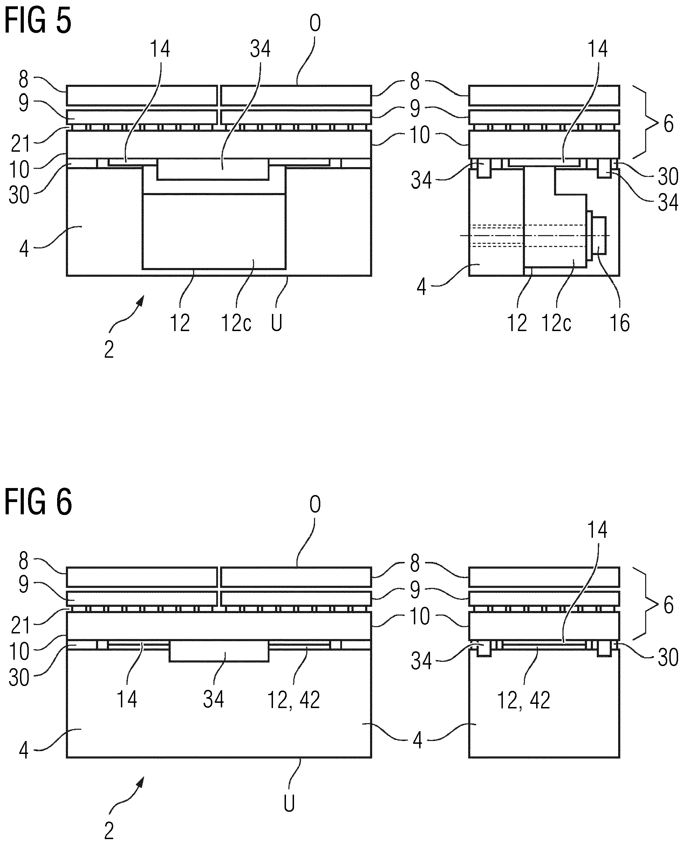

Fig. 5 zeigt eine weitere Ausführungsform, bei welcher das wärmeleitende Metallelement 12 durch einen blockartigen Einsatz 12c gebildet wird. Dieser drückt von der Seite an den Modulträger 4 und wird durch eine Schraube 16 in Position gehalten. Alternativ zur Schraube können auch andere Verbindungstechniken, beispielsweise mit einer Klammer, zum Einsatz kommen, um das Metallelement 12 in Kontakt zu der Unterseite des Substrats bzw. zu dem dort optional aufgebrachten metallischen Belag 14 zu halten. Auch bei dieser Ausführungsform wird das Röntgenkonverterelement 6 durch einen Klebstoff 30 auf dem Modulträger 4 gehalten.

Fig. 6 zeigt eine noch weitere Ausführungsform, bei welcher das Metallelement 12 durch eine Schicht 42 zwischen dem Substrat 10 und dem Modulträger 4, bzw. einem optionalen metallischen Belag 14, realisiert ist. Dabei kann es sich um ein Lot handeln. Das Lot kann vor dem Verkleben des Röntgenkonverterelements 6 auf dem Modulträger 4 in den Spalt 11 eingebracht werden. Nach dem Verkleben kann das Lot dann aufgeschmolzen werden, beispielsweise durch Erhitzen oder Laser. Alternativ kann die Schicht 42 auch durch eine reaktive Lotschicht gebildet werden, die dann lediglich gezündet werden muss und durch eine exotherme Reaktion keine weitere Wärmezufuhr benötigt. Auch dadurch ist eine direkte Metall-zu-Metallverbindung zwischen der Unterseite des Röntgenkonverterelements und dem Modulträger 4 möglich. Ein Metallbelag 14 auf der Unterseite des Substrats ist bei dieser stoffflüssigen Verbindung nicht notwendig.

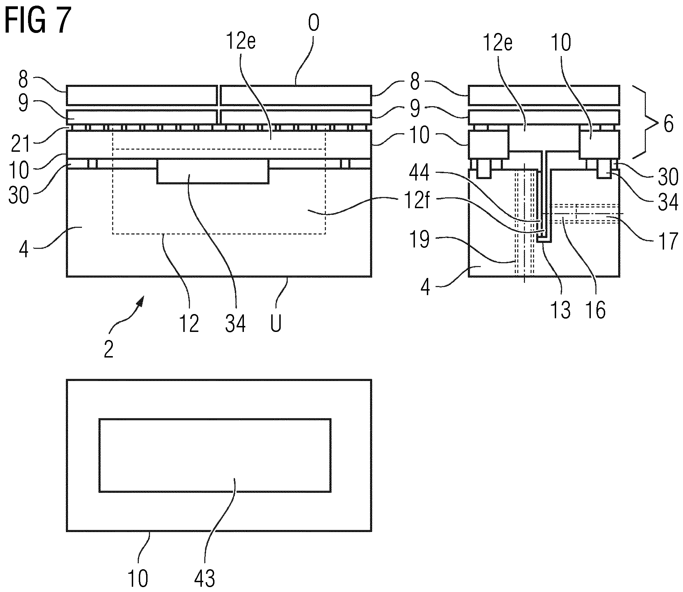

Fig. 7 zeigt eine weitere Ausführungsform, wobei hier neben den Schnittabbildungen auch eine Aufsicht auf das Substrat 10 ohne die elektrischen Verbindungsstellen der Schicht 21 dargestellt ist. Bei dieser Ausführungsform hat das Metallelement 12 über den Bereich 12e einen direkten Kontakt zur A/D-Wandlerschicht 9, und ist somit näher an den Wärmequellen in der Röntgendetektorschicht 8,9. Dies wird dadurch erreicht, dass das Substrat 10 eine durchgängige Ausnehmung 43 aufweist, durch die das Metallelement 12 hindurch reicht.

Dadurch kann die Wärme noch effizienter abgeführt werden, da sie näher an den Wärmequellen vom Metallelement 12 aufgenommen werden kann. Das Substrat 10 ist dann ringförmig aufgebaut. Das Metallelement 12 kann ähnlich ausgebildet sein wie in der Ausführungsform der Fig. 3, also als ein T-förmiges Metallelement, dessen einer Schenkel 12e auf die Unterseite der A/D-Wandlerschicht drückt und dessen anderer Schenkel 12f in einer Ausnehmung 13 des Modulträgers 4 aufgenommen ist.

Das Metallelement 12 wird durch Madenschrauben 16, welche in Gewindebohrungen 17 und 19 eingeführt werden, an den entsprechenden Flächen, also an die Unterseite der A/D-Wandlerschicht 9 und an die Seitenwand der Ausnehmung 13 angedrückt, um einen Metall-zu-Metalloberflächenkontakt sicherzustellen. Alternativ zu den Madenschrauben 16 kann auch ein Reaktivlot eingesetzt werden. Weiterhin kann auch hier ein metallischer Belag auf der A/D-Wandlerschicht 9 vorgesehen sein, um eine weiter verbesserte Wärmeleitung zu erreichen.

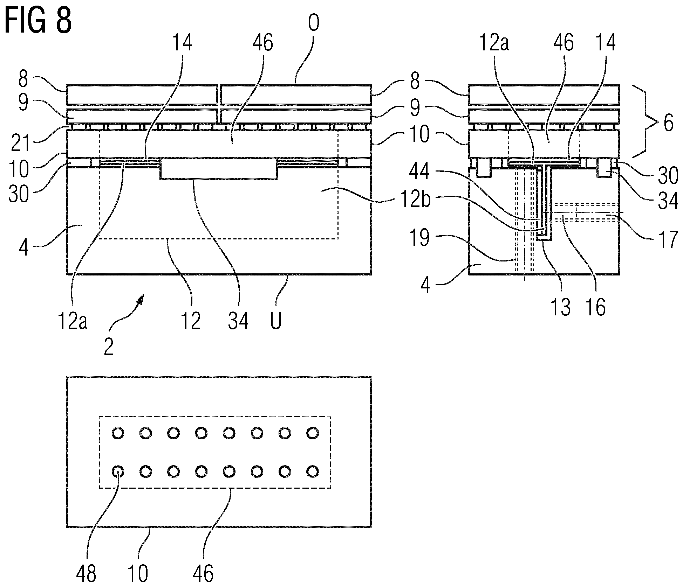

Fig. 8 zeigt eine weitere Ausführungsform, welcher der Ausführungsform der Fig. 3 ähnlich ist, da auch hier ein T-förmiges Metallelement 12 mit Schenkeln 12a, 12b verwendet wird, welches in einer Ausnehmung 13 im Modulträger 4 eingesetzt ist. Bei dieser Ausführungsform hat das Substrat 10 jedoch einen wärmeleitenden Bereich 46, in diesem Fall befindet sich dieser in der Mitte des Substrats.. Die untere Darstellung in Fig. 8 zeigt die Basisplatte 10, ohne die elektrischen Verbindungsstellen der Schicht 21. Durch die thermisch leitende Schicht 46 wird der Wärmefluss zwischen den Sensorelementen 8 und dem Modulträger 4 weiter verbessert. Dies kann beispielsweise dadurch gewährleistet werden, dass in dem thermisch leitenden Bereich 46 zusätzliche Metallschichten und/oder metallisierte Durchgangslöcher 48 vorhanden sind.

Unabhängig vom grammatikalischen Geschlecht eines bestimmten Begriffs sind Personen mit männlicher, weiblicher oder andere Geschlechteridentität mit umfasst.

## Patentansprüche

1. CT-Detektormodul (2), umfassend zumindest ein Röntgenkonverterelement (6), welches eine Oberseite und eine Unterseite aufweist, wobei das Röntgenkonverterelement (6) an seiner Oberseite (O) eine Röntgendetektorschicht (8,9) aufweist und an seiner Unterseite auf einem wärmeleitenden Modulträger (4) befestigt ist, **dadurch gekennzeichnet, dass** das Röntgenkonverterelement (6) zu zumindest einem Metallelement (12) in wärmeleitendem Kontakt steht, und dass das zumindest eine Metallelement (12) in wärmeleitendem Kontakt zu dem Modulträger (4) steht.

2. CT-Detektormodul (2) nach Anspruch 1, **dadurch gekennzeichnet, dass** die Unterseite des Röntgenkonverterelements (6), welches in wärmeleitendem Kontakt zu dem zumindest einen Metallelement (12) steht, zumindest zum Teil mit einem metallischen Belag (14) versehen ist.

3. CT-Detektormodul (2) nach Anspruch 1 oder 2, **dadurch gekennzeichnet, dass** das zumindest eine Metallelement (12) ein flaches Metallelement, insbesondere ein Metallblech, aufweist, welches in einem ersten Bereich (12a) an der Unterseite des Röntgenkonverterelements (6) anliegt und an einem zweiten Bereich (12b) am Modulträger (4) anliegt.

4. CT-Detektormodul (2) nach einem der vorherigen Ansprüche, **dadurch gekennzeichnet, dass** das zumindest eine Metallelement (12) ein Federelement aufweist, welches durch eine Rückstellkraft an die Unterseite des Röntgenkonverterelements (6) gedrückt wird.

5. CT-Detektormodul (2) nach einem der vorherigen Ansprüche, **dadurch gekennzeichnet, dass** das zumindest eine Metallelement (12) durch ein Befestigungsmittel (16) an dem Modulträger (4) befestigt ist.

6. CT-Detektormodul (2) nach einem der vorherigen Ansprüche, **dadurch gekennzeichnet, dass** das zumindest eine Metallelement (12) zumindest ein Befestigungsmittel (18) umfasst, welches am Modulträger (4) festgelegt ist und gegen einen Metallischen Belag (14) auf der Unterseite des Röntgenkonverterelements (6) oder gegen ein an der Unterseite des Röntgenkonverterelements (6) angeordnetes flächiges Metallelement vorgespannt ist.

7. CT-Detektormodul (2) nach einem der vorherigen Ansprüche, **dadurch gekennzeichnet, dass** das zumindest eine Metallelement (12) in eine Ausnehmung (13) im Modulträger (4) eingesetzt ist oder an einer Seitenwand des Modulträgers (4) befestigt ist.

8. CT-Detektormodul (2) nach einem der vorherigen Ansprüche, **dadurch gekennzeichnet, dass** das zumindest eine Metallelement (12) ein Lot oder Reaktivlot (42) umfasst, welches die Unterseite des Röntgenkonverterelements (6) wärmeleitend mit dem Modulträger (4) verbindet.

9. CT-Detektormodul (2) nach einem der vorherigen Ansprüche, **dadurch gekennzeichnet, dass** das Röntgenkonverterelement an seiner Unterseite eine Basisplatte (10) aufweist, welche eine Ausnehmung (43) aufweist, durch welche das zumindest eine Metallelement (12) direkt mit der Röntgendetektorschicht (8, 9) in wärmeleitendem Kontakt steht.

10. CT-Detektormodul (2) nach einem der vorherigen Ansprüche, **dadurch gekennzeichnet, dass** das Röntgenkonverterelement (6) an seiner Unterseite eine Basisplatte (10) aufweist, wobei die Basisplatte (10) zumindest in dem Bereich (46), in welchem sie mit dem zumindest einen Metallelement (12) in wärmeleitender Verbindung steht, Wärmebrücken (48) zwischen der Unterseite des Basisplatte und der Röntgendetektorschicht (8,9) aufweist.

11. CT-Detektormodul (2) nach einem der vorherigen Ansprüche, **dadurch gekennzeichnet, dass** das Röntgenkonverterelement (6) an seiner Unterseite eine Basisplatte (10) aufweist, welche plattierte Durchgangslöcher (48) aufweist, welche Wärmebrücken zwischen der Unterseite des Basisplatte und der Röntgendetektorschicht (8,9) bilden.

12. CT-Detektormodul (2) nach einem der vorherigen Ansprüche, **dadurch gekennzeichnet, dass** der Modulträger (4) Kühlrippen aufweist.

13. CT-Gerät (1), umfassend zumindest ein CT-Detektormodul (2) nach einem der vorhergehenden Ansprüche.
